(19) Europäisches Patentamt
European Patent Office
Office européen des brevets

(11) Publication number: **0 133 756**
**A2**

# (12) EUROPEAN PATENT APPLICATION

(21) Application number: 84304662.4

(22) Date of filing: 06.07.84

(51) Int. Cl.⁴: **C 12 N 15/00**
C 12 N 1/20
//(C12N1/20, C12R1/125)

(30) Priority: 06.07.83 US 511198
08.06.84 US 618902

(43) Date of publication of application:
06.03.85 Bulletin 85/10

(84) Designated Contracting States:
AT BE CH DE FR GB IT LI LU NL SE

(71) Applicant: GENEX CORPORATION
6110 Executive Boulevard
Rockville Maryland 20852(US)

(72) Inventor: Nagarajan, Vasantha
1731 Evlyn Drive
Rockville, MD 20852(US)

(72) Inventor: Banner, Carl D. B.
9925 Dickens Avenue
Bethesda, MD 20814(US)

(72) Inventor: Rhodes, Craig Stuart
3113 Adams Mill Road
Washington, DC 20010(US)

(74) Representative: Holmes, Michael John et al,
Frank B. Dehn & Co. Imperial House 15-19 Kingsway
London WC2B 6UZ(GB)

## (54) Vector for expression of polypeptides.

(57) A replicable plasmidic expression vector, capable of high levels of expression and secretion of polypeptides in a bacillus is disclosed. The vector contains a DNA sequence comprising the promoter and regulatory regions which control expression and secretion of proteases in a bacillus operably liked to a DNA sequence encoding the amino acid sequence of a polypeptide. The expression vector is particularly useful in the production of *B. amyloliquefaciens* proteases in *B. subtilis*. The method facilitates the isolation of proteases from various bacilli.

EP 0 133 756 A2

0133756

# VECTOR FOR EXPRESSION OF POLYPEPTIDES

This invention relates to the construction, isolation and identification of plasmids which contain DNA sequences directing the expression and secretion of specific classes of proteases and which facilely transform microorganisms to hyperproduce and secrete the specified proteases or other proteins.

Proteases are a group of enzymes which hydrolyze the peptide bonds of proteins. Proteases which are produced by bacteria can be classed in two general types. Those which are active at neutral pH and usually require a cofactor, such as zinc, to be active are called neutral proteases and can be inactivated by chelating agents such as ethylenediaminetetraacetic acid (EDTA) which remove the cofactor from the enzyme. Those which are active at high pH and cleave peptide bonds by a process analogous to alkaline hydrolysis are termed alkaline proteases. Alkaline proteases are also referred to as subtilisin and serine protease. Alkaline proteases can be inactivated by chemicals such as phenylmethylenesulfonyl fluoride (PMSF) or by diisopropyl fluorophosphate (DFP). Bacterial proteases are available commercially from a number of suppliers (OPD Chemical Buyers Directory, A.P. Kavaler, ed, Schnell Publishing Co., Inc. New York, New York 1982) and are used industrially to clarify beer, tan leather, tenderize meat, curdle milk and in the formulation of specialized detergents. Bacterial enzymes are extracted from cultures of specific strains of microorganisms, many of which are the result of the careful selection and of artifical mutation (e.g. UV irradiation). Limitations on the production of enzymes include the rate at which the microorganism produces the enzyme, the degree to which the microorganism excretes the enzyme produced, and the stability of the microorganism in culture.

In recent years, processes have been developed for inserting into a bacterial organism a gene or genes from another organism so that the bacterium will produce "foreign" proteins. The technique for introducing the DNA which directs ("codes for") the production of an enzyme involves the cleavage of DNA from the source cell using one of a number of "restriction endonucleases", attaching ("ligating") the cleaved DNA to DNA from a plasmid to form a "vector," and introducing the vector into the host under conditions which make the "transformation" successful.

The organisms of the genus Bacillus are aerobes and have been widely used in the fermentation industry because they are non-pathogenic and, in the case of protein production, because they have the ability to secrete proteins. Most research into recombinant DNA has been performed using E. coli as the host. E. coli is generally a non-secreting microorganism well-developed for the cloning of recombinant plasmids but not well suited for the commercial production of proteins. Bacillus species, particularly B. subtilis, cannot be transformed using monomer plasmid or conventional techniques developed for E. coli. (Canosi et al. Mol. gen. Genet., 166:259 [1978], Keggins et al., Proc. Nat. Acad. Sci. U.S., 75:1423 [1978] and Michel et al., Gene, 12:147 [1978]). A number of alternative strategies have been developed for the transformation of B. subtilis, including the use of plasmid multimers (Canosi), the use of polyethylene glycol to induce DNA uptake in protoplasts (Chang S. and Cohen S.N., Mol. gen. Genet., 168:111 [1979]) and the use of the marker rescue technique (Gryczan et al., Mol.

gen. Genet., 177:459 [1980]). A structural gene for alpha-amylase, including the associated control regions, from B. amyloliquefaciens has been shotgun cloned into B. subtilis. The alpha-amylase was expressed in B. subtilis at a rate five times that produced by the source B. amyloliquefaciens (Palva I. Gene, 19:81-87 [1982]).

A Bacillus transformant, e.g. a B. subtilis transformant, expressing large amounts of proteases would have particular commercial importance. Moreover, an efficient expression vector for transforming B. subtilis which includes a promoter, operator and ribosome binding site, as well as the structural gene for a specific enzyme such as a protease would be useful not only because it could be used to transform B. subtilis to produce the enzyme, but also because it could provide an efficient promoter and regulatory region which could be used for the expression and secretion of heterologous proteins in B. subtilis.

The present invention is based upon applicant's discovery that the genetic information which serves to regulate the expression of proteases in a first bacillus species such as B. amyloliquefaciens can be incorporated into a second bacillus species such as B. subtilis in the form of a replicable plasmidic expression vector to produce a transformant microorganism which is capable of high levels of expression and secretion of the protease or, if desired, other heterologous polypeptides, i.e., polypeptides not naturally produced by the bacillus host.

According to the invention, there is provided a replicable plasmidic expression vector capable of directing high level expression and secretion of a polypeptide in a bacillus transformed therewith, such as B. subtilis, which comprises:

(a) a replicon;

(b) a DNA sequence comprising the promoter and regulatory regions which control expression and secretion of a bacillus protease, such as a B. amyloliquefaciens protease; and

(c) a DNA sequence encoding the amino acid sequence of a polypeptide, operably linked to said promoter and regulatory region.

In one embodiment of the invention, the polypeptide which is encoded is the protease whose expression and secretion in its cell of origin is controlled by the particular promoter and regulatory regions, for example, the promoter and regulatory regions for the alkaline protease or neutral protease genes of B. amyloliquefaciens.

In another embodiment, the polypeptide which is encoded is a heterologous polypeptide which is not normally under the control of the protease promoter and regulatory region. The heterologous polypeptide can be a prokaryotic protein, e.g., protein A, or it can be a eukaryotic protein, e.g., prorennin.

There are also provided, in accordance with the invention, transformant microorganisms containing the previously mentioned replicable plasmidic expression vector; a method of producing a polypeptide by expression of the DNA encoding the polypeptide in the transformant microorganism; and a method of producing a microorganism for the expression of a protease enzyme which comprises transforming a bacillus, such as B. subtilis, with the replicable plasmidic expression vector of the invention, preferably a vector in which the polypeptide which is encoded by the DNA sequence is a B. amyloliquefaciens protease.

In the accompanying drawings,

Fig. 1 represents a partial restriction map of the plasmid pGX2110 which was produced by inserting into pBD64 an insert encoding a promoter and regulatory region, as well as the associated structural gene for alkaline protease (apr[BamP]) derived from B. amyloliquefaciens. In Fig. 1, ▬▬ denotes apr[BamP], /\/\/\ indicates other B. amyloliquefaciens DNA, and ——— indicates pBD64 vector sequence. A detailed map of the insert indicating the location of several restriction sites and of apr[BamP] (———>) is shown beneath the plasmid.

Fig. 2 is a partial restriction map of the plasmid pGX2109 which was produced by inserting into pBD64 an insert encoding a promoter and regulatory region, as well as the associated structural gene for neutral protease (npr[BamP]) derived from B. amyloliquefaciens. In Fig. 2, ▬▬ denotes npr[BamP], /\/\/\ indicates other B. amyloliquefaciens DNA, and ——— indicates pBD64 vector sequence. A detailed restriction map of the insert, indicating the location of several restriction sites and of npr[BamP] (———>) is shown beneath the plasmid.

Fig. 3 depicts the nucleotide sequence of the apr[BamP] gene and the encoded amino acid sequence of alkaline protease (subtilisin) produced by B. amyloliquefaciens. Both strands were sequenced from several independent overlapping clones. The putative ribosome binding site and the transcriptional terminator have been underlined. The previously published amino acid sequences (Smith et al., J. Biol. Chem., 214:5974-5976 [1966]) at the regions of mismatch (indicated in Fig. 3 by asterisks) are: 56,57(Pro,Asn); 61(Asp); 88,89(Ser,Ala); 98,99(Asp,Ala); 158,159(Ser,Thr); 251(Gln).

Fig. 4 depicts the nucleotide sequence of the npr[BamP] gene and the encoded amino acid sequence of neutral protease produced by B. amyloliquefaciens. Both strands were sequenced from several independent overlapping clones. The putative ribosome binding site and the transcriptional terminator have been underlined. The N-terminus of the mature protein has been identified based on perfect homology to the N-terminal sequence of neutral protease A from B. subtilis NRRL B3411 (Levy et al., Proc. Nat'l. Acad. Sci., U.S.A., 72:4341-4345 [1975]).

Fig. 5 is a graphic illustration of the construction of plasmid pGX2136 which contains a portion of the gene coding for protein A (S. aureus) fused in-frame to the 3' end of the signal sequence of apr[BamP].

Fig. 6 is a graphic representation of the construction of plasmid pGX2140 which contains a portion of the gene coding for protein A (S. aureus) fused in-frame to the 3' end of the signal sequence of npr[BamP].

The replicable plasmidic expression vector of the invention is produced, using techniques of DNA recombination, by inserting a DNA sequence described hereinafter into a plasmid which is capable of transforming and replicating in a host microorganism. (Unless otherwise indicated, all DNA referred to herein is in the form of double stranded DNA.) A plasmid is a non-chromosomal loop of double stranded DNA found, often in multiple copies per cell, in microorganisms. The plasmid contains within its DNA sequence the genetic information necessary for replicating itself (i.e. a "replicon"). Additionally, many plasmids contain sequences of DNA which encode a phenotypic trait, such as resistance to specific antibiotics, which is useful when one desires to screen for the presence of the particular plasmid.

Any plasmid which is capable of transforming and replicating itself in the host microorganism can be employed in the practice of this invention. When the host microorganism is B. subtilis, we prefer to employ the plasmid pBD64 to prepare the replicable plasmidic expression vector and to use, as the host, a B. subtilis containing the homologous plasmid pUB110. Both plasmids are described in Gryczan et al., supra. An homologous plasmid is a resident plasmid in B. subtilis which has a large region of DNA sequence homology with the exogenous plasmid which one desires to introduce and which is capable of recombining with the exogenous plasmid during transformation. The resident plasmid pUB110 is an homologous plasmid for pBD64. The use of a resident homologous plasmid to increase the efficiency of transformation -- which we employed in practicing the invention -- is described in the Gryc- et al. paper and is known as the marker rescue transformaton technique.

The DNA sequence which is inserted into the plasmid to produce the replicable plasmidic expression vector of the invention contains a DNA sequence comprising the promoter and regulatory regions which control expression and secretion of a protease in a first bacillus organism, preferably B. amyloliquefaciens, operably linked to a DNA sequence encoding the amino acid sequence of a polypeptide. In one embodiment, the encoded polypeptide is a B. amyloliquefaciens protease (alkaline or neutral).

The promoter is a sequence of deoxyribonucleotides which is recognized and bound by the enzyme RNA polymerase and may be overlapped by the so-called operator sequence. The operator sequence is recognized and bound by a repressor protein, which is not present or is inactive under conditions of expression. The RNA polymerase binds to the promoter and then travels along the coding strand of the DNA (provided it is not blocked by a bound repressor protein) transcribing the sequence of bases into corresponding mRNA. A portion of the DNA which is transcribed encodes a ribosome binding site, known as the Shine-Dalgarno sequence. This is followed by a translation initiation signal, normally ATG in the DNA sequence, which is the nucleotide triplet encoding the first amino acid in the structural gene. The rest of the transcript encodes the structural gene of a polypeptide. RNA polymerase action ceases when it encounters a termination signal in the DNA. The resulting mRNA can be effectively translated at the ribosome to produce the desired protein. In the

case of secreted proteins, such as α-amylase and proteases, in addition to the promoter, operator and ribosome binding site there is also a "signal sequence" following the translation initiation signal. This encodes a polypeptide of about 15 to 30 amino acids in length that usually contains a positively charged amino terminal domain followed by a hydrophobic domain. The signal sequence, which precedes the amino acid sequence of the mature protein, is necessary for secretion of proteins across the cell membranes and is removed from the secreted protein during or immediately after translocation across the cell membrane.

The promoter, operator, ribosome binding site and translation initiation sequences serve to control the efficiency of expression of the protein. As used herein, the term "promoter and regulatory regions" refers to the DNA sequence containing the bases encoding the promoter, operator, ribosome binding site and translation intitiation signal, as well as the secretion signal sequence.

The DNA sequence (promoter and regulatory region) which is incorporated into the replicable plasmidic expression vector of the invention preferably is one which controls the expression and secretion of a protease in *B. amyloliquefaciens* and, therefore, can be obtained from *B. amyloliquefaciens*. In one embodiment of the invention, the structural gene which is operably linked to the promoter and regulatory regions (including the signal sequence) is one which encodes the amino acid sequence of the protease whose expression and secretion is controlled by the particular promoter and regulatory sequence in *B. amyloliquefaciens*. It will be appreciated, however, that DNA sequences encoding other useful heterologous polypeptides can be operably linked to the promoter and regulatory regions derived from *B. amyloliquefaciens*, using known techniques of *in vitro* DNA recombination, and inserted into a plasmid to produce a replicable plasmidic expression vector which can be employed to direct expression and secretion of the heterologous polypeptide in *B. subtilis*. As merely exemplary of such useful polypeptides one can mention eukaryotic polypeptides such as human interferons, insulin, human and animal growth hormones, prorennin and the like, and prokaryotic polypeptides such as protein A. These are examples of polypeptides having known amino acid sequences which have been expressed by recombinant DNA techniques in *E. coli*. An efficient means of expressing these polypeptides in *B. subtilis* such as the method of our invention offers distinct advantages over expression in *E. coli*, for example, secretion of the polypeptide from the organism, which simplifies purification and increases yield, and elimination of the danger of contamination of the product by *E. coli* endotoxins.

The DNA sequence encoding the promoter and regulatory regions and, if desired, the DNA sequence encoding the *B. amyloliquefaciens* protease can be obtained by direct cleavage of a segment containing the desired sequence from chromosomal DNA of *B. amyloliquefaciens*.

Alternatively, one could isolate the mRNA from *B. amyloliquefaciens* which is transcribed from the desired DNA sequence and use the mRNA as a template to produce cDNA in the presence of reverse transcriptase. The cDNA can then be employed to isolate the desired promoter, regulatory regions and structural gene.

The chromosomal DNA or the cDNA can be inserted into a cloning vector which can be used to transform a host microorganism. The transformant is then grown up to produce a large population of clones containing the desired DNA sequence.

In order to insert the Bacillus DNA, e.g. the B. amyloliquefaciens DNA into the plasmid, it is necessary that the DNA have ends which are complementary to the ends generated at an endonuclease cleavage site on the plasmid conveniently located for expression. This can be achieved by digesting, either completely or partially, the ends of the DNA segment from B. amyloliquefaciens with a restriction endonuclease corresponding to the desired insertion site on the plasmid. The DNA sequences encoding the promoter and regulatory regions and the amino acid sequence of the polypeptide are inserted into the plasmid (which has been linearized by cleavage with an appropriate restriction endonuclease) by reacting in the presence of a ligating enzyme such as T4 DNA ligase.

The resulting recombinant plasmids are used to transform the host bacillus microorganism, which can be screened for the presence of the expression vector having the desired DNA sequence in the appropriate orientation for expression. Transformation can be effected using known techniques (Contente, S. and Dubnau, D., Plasmid, 2:555-571(1979) and Chang & Cohen, supra).

The transformants can be screened for the presence of the desired recombinant plasmid by assaying for the presence of the activity characteristic of the polypeptide encoded by the DNA sequence. In the case of the DNA sequence encoding a B. amyloliquefaciens protease, the presence of the desired recombinant can be determined by the ability of the transformant cells to clear skim milk in agar.

If desired, the replicable plasmidic expression vector can be removed from the screened transformant by known techniques, such as the boiling preparation method, and used to transform other host bacillus microorganisms or one can employ the screened transformant directly to express the polypeptide. In the event one wishes to express the polypeptide in *E. coli*, it is preferred to employ a "shuttle vector," which can be prepared by cleaving and recombining the replicable plasmidic expression vector with a plasmid which is capable of efficient replication in *E. coli*, e.g., the plasmid pBR322.

Since *E. coli* is an excellent host for cloning, one may desire to prepare a shuttle vector from the replicable plasmidic expression vector for the purpose of replicating it in *E. coli*. The cloned vector can then be isolated from the *E. coli*, and the replicable plasmidic expression vector can be recovered therefrom by cleavage and recircularization and used to retransform *B. subtilis* or another host for expression.

The replicable plasmidic expression vector of the invention can be used to transform stage zero sporulation mutants of *B. subtilis*. The use of stage zero sporulation mutants of *B. subtilis* in fermentations offers the theoretical advantage of better control of contamination of the environment. Existing stage zero sporulation strains produce very low levels of protease. The plasmids produced and isolated by the above-described procedure are readily introduced into stage zero sporulation mutants of *B. subtilis* using the protoplast transformation method used to transform other strains of *B. subtilis*. The transformed stage zero sporulation *B. subtilis* produced the specified proteases at rates qualitatively indistinguishable from other transformed *B. subtilis*. The cells retain their characteristic inability to produce spores.

The polypeptide encoded by the DNA sequence in the replicable plasmidic expression vector can be produced by growing up the transformed microorganism under known fermentation conditions until a desired cell density is achieved and subjecting the transformants to conditions which initiate expression. Addition of inexpensive carbon sources such as soy protein, fish meal or any denatured protein that is available and economical may enhance expression.

The expressed polypeptide is then recovered from the other proteins and contaminants in the fermentation broth and purified using known techniques such as size exclusion chromatography, immunoaffinity chromatography and the like.

In one embodiment of the invention, a replicable plasmidic expression vector encoding B. amyloliquefaciens protease can be produced and used to transform B. subtilis in the following manner.

Cellular DNA from B. amyloliquefaciens is isolated using standard techniques and partially digested with a restriction endonuclease such as MboI under very mild conditions. It is then ligated to a vector, e.g., to the BamHI site of pBD64 which has ends complementary to those left by the MboI digestion. In the preferred embodiment, the B. amyloliquefaciens DNA was partially digested with MboI and ligated to the BamHI site of pBD64, which has markers for chloramphenicol and kanamycin resistance. An homologous plasmid to pBD64 is pUB110. (Other combination of homologous plasmids and appropriate restriction endonucleases are suitable, provided that any cleavage sites in the B. amyloliquefaciens DNA within the desired insert are not cut.)

Transformed cells expressing protease are identified by growing the cells on agar containing skim milk. Colonies which produce zones of clearing of the milk are streaked for single colonies which clear milk at a greater rate than control colonies of B. subtilis and the desired colonies are easily selected and grown in shaker flasks. When the plasmid markers are for antibiotic resistance, the antibiotic may be added to the growth medium to select against colonies not carrying the plasmid. When pBD64 is used as the plasmid, chloramphenicol is the preferred antibiotic. Clones exhibiting the selection characteristic are grown for further analysis. Plasmids containing the genes for protease expression are isolated from cell cultures by standard techniques, such as the boiling preparation method (Holmes, D. S and M. Quigley, Anal. Biochem., 114-193 [1981]). The isolated plasmids will readily retransform B. subtilis to express both the protease and the selected markers (i.e. antibiotic resistance). Alternatively, the isolated plasmids may be cloned in a host such as E. coli after being cleaved and ligated to a suitable vector for the transformation of E. coli. such as the previously mentioned shuttle vector. When such a shuttle vector is produced, the ligated fragments may orient in either direction and it is desirable to screen the cloned plasmids at some point to select out the non-functional orientations.

B. amyloliquefaciens contains structural genes encoding both neutral and alkaline protease. B. subtilis transformed as described above will express either protease type, depending upon the particular structural gene incorporated into the replicable plasmidic expression vector. When vectors encoding the production of B. amyloliquefaciens protease were prepared in this manner, two different plasmids encoding alkaline protease and one plasmid for neutral protease were isolated. On the basis of restriction mapping of the isolated plasmids, the difference between the two alkaline protease plasmids appears to be the inclusion of an additional untranslated six hundred base pair sequence at the 3' end of the structural gene encoding the protease. No other functional or chemical differences are apparent and both proteins were approximately equal in activity. It is to be expected that minor, non-functional differences in plasmids and proteases may be obtained when this technique is used to produce the replicable plasmidic expression vectors and transformant microorganisms.

The replicable plasmidic expression vectors produced as described herein were compared for their ability to direct the hyperproduction of protease in B. subtilis. The rate of production was compared to that of B. subtilis transformed with the original plasmid pBD64 and with the B. amyloliquefaciens strain from which the DNA inserts were obtained. The rate of digestion of a dye-protein complex such as hide powder azure provides a convenient method for comparison. Under identical conditions, B. subtilis strains transformed with the plasmids containing the DNA insert from B. amyloliquefaciens were 150 to 200 times more productive of proteases than B. subtilis transformed with pBD64, and 1.5 to 2 fold more productive than B. amyloliquefaciens. Neutral protease producing cultures were marginally more productive than alkaline protease producing transformants in this test.

In another embodiment of the invention, a replicable plasmidic expression vector is constructed in which a heterologous structural gene encoding a polypeptide other than a B. amyloliquefaciens protease is placed under the control of the promoter and regulatory region of the alkaline protease (apr[BamP]) or neutral protease (npr[BamP]) gene from B. amyloliquefaciens. The expression vector is used to transform B. subtilis, in which it directs expression and secretion of the heterologous protein.

We have isolated and sequenced both apr[BamP] and npr[BamP]. The DNA sequences and inferred amino acid sequences of alkaline protease and neutral protease from B. amyloliquefaciens are respectively illustrated in Fig. 3 and Fig. 4. In both cases, DNA sequencing indicated a large open reading frame preceding the sequence encoding the mature protease. The inferred amino acid sequence of each gene contained a signal sequence and an additional polypeptide sequence ("pro" sequence) preceding the mature protein. We have identified the start point of translation as amino acid residue -107 for apr[BamP] and -221 for npr[BamP]. To demonstrate that the start point of translation of apr[BamP] in vivo is codon -107, codon -103 (AAA) was changed to an ochre (TAA) by site directed mutagenesis (Zoller and Smith, Nuc. Acids Res., 10:6487-6500 [1982]). Alkaline protease was produced from this ochre mutant derivative of apr[BamP] only when the host strain was $Su^+$.

An expression vector of the invention capable of directing expression and secretion of a heterologous polypeptide in B. subtilis can be constructed by inserting the structural gene for the heterologous protein into apr[BamP] or npr[BamP] downstream from and in phase with the coding sequence of the signal peptide. The hybrid DNA coding for the signal peptide of the B. amyloliquefaciens protease fused to the heterologous polypeptide, on an appropriate plasmid, is used to transform B. subtilis. A fusion protein comprising the signal peptide and the heterologous polypeptide will be expressed and secreted from the B. subtilis, with attendant cleavage of the signal peptide to release the heterologous polypeptide into the surrounding medium. If any portion of the sequence of apr[BamP] or npr[BamP] coding for the "pro" region of the protease is left intact upstream from the gene coding for the heterologous polypeptide, then that encoded portion of the "pro" region will remain fused to the N-terminus of the secreted polypeptide. Since "pro" regions have previously been associated only with eukaryotic proteases, it was somewhat surprising to find pro-coding regions in apr[BamP] and npr[BamP]. Moreover, the possible role of the "pro" region in facilitating secretion or protecting the protein from degradation during secretion was uncertain. Consequently, it was an unpredictable finding that secretion of the expression product of a heterologous gene linked directly to the signal coding sequence of apr[BamP] or npr[BamP], i.e., without the "pro" region, could be effected in B. subtilis. The putative signal processing site between the pre and pro regions has been identified based on consensus' "signal sequence" outlined by Perlman and Halvorson (J. Mol. Biol., 167:391-409 [1983]). The signal processing site can be determined by sequencing the amino terminus of the secreted protein. If the signal processing is found ustream or downstream of the proposed signal, heterologous fusions can be accordingly modified.

Insertion of the structural gene for the heterologous polypeptide downstream from the signal peptide coding region is advantageously effected by employing oligonucleotide-directed mutagenesis (Norris et al., Nuc. Acids Res., 11:5103-5112 [1983]) of the apr[BamP] or npr[BamP] to create an endonuclease cleavage site just downstream from the signal peptide coding region which is compatible with a cleavage site on the gene for the desired heterologous polypeptide. For example, we created a BamHI cleavage site just downstream from the signal coding sequence of apr[BamP] by the insertion of six deoxyribonucleotides after the fifth deoxyribonucleotide of the pro-coding region as follows:

Apr[BamP] . . . GCA GGG . . .

↑

Insert GGATCC

Mutated Sequence . . . GCA GGG GAT CCG . . .

BamHI

The mutated apr[BamP] gene was cleaved at the BamHI site and ligated to a compatible BclI site 22 codons into the structural gene for protein A from S. aureus to create a fused gene coding for the signal peptide and first two amino acids of the pro region of alkaline protease (coded by GCA GGG) followed by the protein A sequence beginning with amino acid 23. A plasmid containing this fused gene was used to transform B. subtilis. Transformants containing the plasmid were grown and were found to secrete a protein which was shown by enzyme-linked immunosorbent assay and immunoblot analysis to correspond immunologically to protein A.

Genes for other heterologous polypeptides can be inserted into apr[BamP] or npr[BamP] and used to secrete the desired polypeptide from B. subtilis in a manner analogous to that which we employed with the protein A gene.

The invention will be further illustrated by the following non-limiting examples:

## Example I

### Construction of a Replicable Plasmidic Expression Vector Containing a Structural Gene For Protease and Transformation of B. subtilis

Bacillus amyloliquefaciens (ATCC 23844) was isolated according to Saito, H. and Miura (Biochim. Biophys. Acta, 72:619 [1963]) and partially digested with MboI (Old, R.W. and Primrose, S.B., "Principles of Gene Manipulation", 2nd ed., Univ. of Calif. Press, Berkley, 1981). 250 μg of B. amyloliquefaciens chromosomal DNA was digested with 192 units of MboI for 7 min. at 37°C. The digestion was terminated by phenol:chloroform extraction and the DNA was ethanol precipitated. Ligation was performed by using chromosomal DNA:pBD64 plasmid at a ratio of 1:1 using 2 μg

DNA/10 µl and adding 1 unit of T4 ligase and incubating at room temperature (~ 25°C.) for 60 min. and was terminated by freezing the DNA at -70°C. B. subtilis strain BR151 harboring pUB110 was transformed with the ligated DNA and plated on Luria broth plus 1.1% agar and 3% skim milk, and incubated at 37°C. After sixteen hours, a lawn of transformants was observed on the direct transfer plate. There were approximately 2,000 colonies on the $10^{-1}$ dilution plate and approximately 240 colonies onto the $10^{-2}$ plate. Zones of clearing of the milk were observed after 17 hours and colonies from the cleared zones were picked and streaked for single colonies. After 24 hours the entire plate cleared due to protease produced by the receipient strain. The single colonies were isolated and grown in Penassay broth with 10 µg/ml chloramphenicol for five hours at 37°C.

## Example II

### Isolation and Identification of Plasmids pGX2109, pGX2110 and pGX2111

The culture was centrifuged and the plasmids were isolated from the cell pellet by the boiling preparation method modified as follows. The cells were resuspended in 350 µl of 8% Sucrose, 5% Triton, 50 mM EDTA 50 mM Tris pH 8.0 (STET), 500 ug lysozyme was added, and the suspension was incubated for 15 minutes at 37°C, then boiled for one minute. The chromosomal DNA and the protein were removed by centrifugation at 12,000 x G for 20 minutes, and 5 ul proteinase K (50 µg/ml) was added to the pellet and incubated for twenty minutes at 37°C. The sample was heated for twenty minutes at 70°C to inactivate the proteinase K. Two volumes of isopropyl alcohol were added to precipitate the DNA and the solution was allowed to stand for 30 minutes at -20°C. The pellet was washed with 70% ethyl

70% ethyl alcohol and dried *in vacuo*. The plasmid DNA was dissolved in 50 µl of distilled water and five ul was used to transform *B. subtilis* (BR151). The transformants were screened for kanamycin and/or chloramphenicol resistance and clearance of milk. Transformed colonies were either kanamycin resistant, chloramphenicol resistant and protease positive or kanamycin resistant, chloramphenicol resistant and protease negative. No protease positive chloramphenicol sensitive colonies were found. Nine independent clones were identified.

The nine clones were single colony purified by streaking onto Luria broth, 1.1% agar and 3% skim milk. The extracellular supernatant from each purified colony was assayed for protease activity in the presence of EDTA or phenylmethylsulfonyl flouride using the procedure of Vasantha and Freese, *J. Bact.*, *144*:1119-1125 (1980). The protease activities of two colonies were inhibited by EDTA to 95% and not inhibited by PMSF. Seven were inhibited by PMSF (95%) and not by EDTA. The former were designated as producing neutral protease, the latter as producing alkaline protease.

Plasmids from each single colony were isolated as previously mentioned. The isolated plasmids were restriction mapped. The colonies producing neutral protease contained one plasmid, labelled pGX2109 which has restrictions sites as shown in Fig. 2. It has been deposited at the USDA Northern Regional Research Laboratory, Peoria, Ill. as B15436. The alkaline protease-producing colonies contain two closely related plasmids labelled pGX2110 and pGX2111. Restriction sites in pGX2110 are illustrated in Fig. 1. Plasmid pGX2111 gave an identical restriction map to pGX2110, except that it contained an additional 600 base pair segment in the *BglII-BamHI/MboI* region at the 3' end of the insert. Plasmid pGX2110 has been deposited at NRRL as B15437.

In order to identify the location of the control region and structural gene in the insert in pGX2110, an eight base pair SalI linker was introduced into various restriction sites to determine its effect on protease production. In separate experiments, the plasmid was either digested with SmaI or HindIII, and BglII and treated with SI nuclease using the conditions suggested by manufacturer. The SalI linker was inserted at the cleavage site using T4 ligase. The plasmid containing the linker was used to transform B. subtilis (Contente and Dubnau, supra). The transformants were assayed for protease activity by their ability to clear skim milk. Introduction of the SalI linker at the SmaI site, the BglII site and HindIII sites did not inactivate the protease gene. The plasmid was then cleaved with ClaI and the large fragment recircularized by ligation in the presence of T4 ligase and used to transform B. subtilis. The transformants assayed negative for protease activity. Based on the results of these experiments, it is clear that the ClaI site is in the gene for apr[Bamp]. The amino acid sequence deduced from the DNA sequence shows extensive homology to the published amino acid sequence of subtilisin (Fig. 3) and the ClaI site was in codon 36 of the mature protein.

## Example III

### Hyperproduction of Protease in B. subtilis Cultures

Plasmids pGX2109 (neutral protease), pGX2110 (alkaline protease) and pBD64 (the plasmid which was used to form the vector for transforming B. subtilis BR151) were used to transform B. subtilis BR151. The transformants as well as B. amyloliquefaciens were grown separately in Penassay broth. Protease activity in the supernatant was measured using hide powder azure as the substrate. B. subtilis transformed by pGX2109 and pGX2110 were essentially equivalent in protease activity after 48 hours and ten-fold more productive of protease than B. amyloliquefaciens. B. subtilis transformed by pBD64 exhibited less than one-tenth of the B. amyloliquefaciens activity and one-one hundredth the activity of B. subtilis transformed by pGX2109 or pGX2110.

## Example IV

### Expression of Protease in Stage Zero Sporulation Mutants of B. subtilis

Protease negative, stage zero sporulation mutants of B. subtilis (strain name IS53,SpOOB,) were transformed by the previously described procedure. Cells were plated on L.B. 1.1% agar and 3% skim milk. Clearance of milk was indistinguishable from pGX2110 in BR151 (a sporulating strain). The replicable plasmidic expression vector is therefore capable of expression in non-protease producing B. subtilis cell lines and in particular, one not normally producing spores. Naturally occuring stage zero sporulating B. subtilis is not found to produce proteases and a correlation is observed between the lack of sporulation and the lack of protease production.

## Example V

### Construction of secretion vectors for Δ22-protein A using apr[BamP] signal sequences

#### Secretion vector using apr[BamP] signal sequence

The entire apr[BamP] gene was cloned in the M13 phage vector M13mp9 (Messing and Viera, Gene, 19:269-276 [1982]) as an EcoRI-SalI fragment from the plasmid pGX2125. Plasmid pGX2125 was produced by subcloning a SmaI-BglII fragment from pGX2110, containing apr[BamP], into pGX2104, where it is present on an EcoRI-SalI fragment. Using the oligonucleotide-directed mutagenesis procedure of Norris et

BAD ORIGINAL

al., (*Nuc. Acids Res.*, *11*:5103-5112 [1983]), a *Bam*HI site was created just after the junction of the signal coding sequence and the prosequence by inserting the sequence GGATCC as follows:

```
                  GGATCC
                    ↓
CAG   GCG { GCA   GGG   AAA
gln   ala { ala   gly   lys
            ↓
                     BamHI
                 ┌─────────────┐
CAG   GCG { GCA   GGG   GAT   CCG   AAA
gln   ala { ala   gly   asp   pro   lys

signal    { pro
```

M13mp9 containing apr[*Bam*P] was grown in YT broth and the single stranded phage DNA was prepared according to Zoller and Smith (*supra*). The mutagenic oligonucleotide 5'TGCCCAGGCGGCAGGGGATCCGAAATCAAACGGGGA 3' and the M13mp9 universal primer 5'GTAAAACGACGGCCAGT 3' were annealed at 20 pmoles and 16 pmoles, respectively to the template DNA (0.8 to 1.0 pmole). Annealing and elongation were carried out according to Norris *et al.* After 2 hours incubation at room temperature, the mutagenic mix was digested with *Eco*RI and *Sal*I and ethanol precipitated using *Eco*RI and *Sal*I digested pGX251 (shuttle vector) as carrier DNA. An aliquot of the precipitated DNA was subject to gel electrophoresis to verify that the fragment of interest (*Eco*RI to *Sal*I fragment) had been successfully synthesized *in vitro*. The rest of the precipitated DNA was ligated and *E. coli* was transformed with the ligated DNA. 24 of the *E.*

coli transformants were screened for the presence of the BamHI site and one was found to contain the BamHI site. That plasmid has been designated as pGX2134 (see Fig. 5), which is capable of replication in both E. coli and B. subtilis. Plasmid pGX2134 can be used for insertion of any heterologous gene having a BamHI-compatible site at its 5' end and to direct expression and secretion of the heterologous gene product in B. subtilis.

Plasmid pGX2912 contains a full-length gene coding for protein A from S. aureus (see Fig. 5). Plasmid pGX2912 was digested (partially) with BclI and with PvuII. The BclI-PvuII fragment contained the entire 3' end of the protein A gene beginning with the codon for amino acid 23 (Δ22-protein A). The BclI sticky end is complementary with sticky end produced by BamHI cleavage.

Plasmid pGX2134 was digested with BamHI and PvuII. The BclI-PvuII fragment coding for Δ22-protein A was ligated to cleaved pGX2134 DNA to produce plasmid pGX2136 (see Fig. 5). Plasmid pGX2136 contains the promoter and regulatory regions of apr[BamP], including the signal peptide coding sequence, fused to the Δ22-protein A gene through two codons, GCA GGG, corresponding to the first two amino acids of the pro-region from apr[BamP]. The sequence of pGX2136 at the fusion of the apr[BamP] and Δ22-protein A coding sequences can be illustrated as follows:

```
                 apr[BamP]                Δ22-protein A
          ┌──────────────────────┐     ┌─────────────────┐
. . . CAG   GCC | GCA   GGG       GAT    CAA . . .
         signal | ala   gly       asp    gln
```

Secretion vector using npr[BamP] signal sequence

An EcoRI-EcoRV fragment from the plasmid pGX2109 containing a portion of the npr[BamP] gene coding for the promoter, signal sequence, pro-region and approximately 60% of the mature protein was cloned in the M13 phage vector M13mp19 (J. Norrander, T. Kempe and J. Messing, *Gene*, *26*:101-106 [1983]) at the EcoRI and SmaI site. M13mp19 containing the npr[BamP] (EcoRI to EcoRV) fragment was highly unstable. Therefore, independent plaques (~ 36) were screened for the phage with the entire insert by agarose gel electrophoresis. Single stranded DNA was made from these and oligonucleotide directed mutagenesis was performed using the method of Norris *et al.*, as follows:

```
                      GGATCC
                         ↓
CAG   GCC | GCT   GAG   AAT
gln   ala | ala   glu   asn
              ↓
                          BamHI
                        ┌─────────┐
CAG   GCC | GCT   GAG   GAT   CCG   AAT
gln   ala | ala   glu   asp   pro   asn
          |
   signal | pro
```

The mutagenic oligonucleotide 5'TGTTCAGGCCGCTCAGGATCCGAATCCTCAGCTTAA 3' (20 pmoles) and the M13 universal primer (12 pmoles) were used with template DNA (0.8 to 1.0 pmoles). The mutagenesis was performed as outlined for apr[BamP]. *E. coli* was transformed and 24 transformants were screened and one had the BamHI site and that plasmid was designated as pGX2138 (see Fig. 6), which is capable of replication in both

E. coli and B. subtilis. Plasmid pGX2138 can be used for insertion of any heterologous gene having a BamHI-compatible site at its 5' end to direct expression and secretion of the heterologous gene product in B. subtilis.

Plasmid pGX2138 was digested with BamHI and EcoRV. Plasmid pGX2912, containing the protein A gene, was digested (partially) with BclI and with PvuII. The BclI-PvuII fragment coding for Δ22-protein A was ligated to the cleaved pGX2138 DNA (EcoRV and PvuII cleavage both generate blunt ends) to produce plasmid pGX2140 (see Fig. 6). Plasmid pGX2140 contains the promoter and regulatory regions of npr[BamP], including the signal peptide coding sequence, as well as two codons, GCT GAG, corresponding to the first two amino acids of the pro-region of npr[BamP]. Restriction analysis of pGX2140 indicated that it had picked up the entire BclI fragment of the protein A gene missing the 22 amino acids at the N-terminus and 45 amino acids at the C-terminus. The sequence of pGX2140 at the junction of the npr[BamP] and Δ22-protein A coding sequences is similar to that illustrated above for the apr[BamP]/Δ22-protein A fusion with the two amino acids being ala and glu at the junction.

## Example VI

## Expression and secretion of Δ22-protein A

Plasmids pGX2136, containing a Δ22-protein A coding sequence under the control of the apr[BamP] promoter and regulatory region, and pGX2140, containing a Δ22-protein A (less 45 C-terminal amino acids) coding sequence under the control of the npr[BamP] promoter and regulatory region, were each used to transform B. subtilis strain 4935 by the procedure of Chang and Cohen (Mol. gen. Genet., 168:111-115 [1979]) and transformants were selected for chloramphenicol resistance. B. subtilis strain 4935 is a low protease derivative strain of B. subtilis BR151. All the chloramphenicol resistant transformants were found to be protein A positive by enzyme-linked immunosorbent assay.

Transformants carrying pGX2136 and pGX2140 were each inoculated into two different culture media (see Table I) and grown for 24 hours. The amount of Δ22-protein A secreted into the medium was determined by the procedure of Lofdhal et al. (Proc. Nat'l. Acad. Sci., 80:697-701 [1983]). Results are presented in Table I. The results show that the promoter and regulatory regions of both the apr[BamP] and npr[BamP] genes were capable of directing expression and secretion of a heterologous protein in B. subtilis.

TABLE I

Amount of Secreted Protein A (mg/l)

| Medium | pGX2136 in GX4935 | pGX2140 in GX4935 |
|---|---|---|
| Medium A[1] | 3 | 710 |
| Synthetic medium[2] | 10 | 60 |

1) Medium A contains (per liter): Tryptone 33 g; Yeast extract 20 g; NaCl 7.4 g; 3M NaOH 12 ml; $Na_2HPO_4$ 8 g; $KH_2PO_4$ 4 g; Casamino acids 20 g; Glucose 10 g; $MnCl_2$ 0.06 mM, and Initial pH 7.5.

2) Synthetic medium as described by Vasantha and Freese, J. Bacteriol., 144:1119-1125 (1980) with 100 mM MOPS replaced by 100 mM potassium phosphate + 1% glucose + 0.2% malate + 1% yeast extract + 1% casamino acids.

## Example VII

### Construction of secretion vectors for prorennin using apr[BamP] and npr[BamP] signal sequences

*E. coli* strain GX1670 contains plasmid pGX2231, which carries a gene coding for a fusion of the first 27 amino acids of the *trp*B expression product and the sequence of prorennin, less its 2 N-terminal amino acids (Δ2-prorennin), linked through a 10-amino acid spacer sequence. Δ2-Prorennin has been shown to have the biological activity of full-length prorennin, i.e., it is capable of undergoing autocatalytic cleavage to produce the active form of the milk clotting enzyme rennin (see copending commonly assigned U.S. Patent Application Serial No. 528,421, the disclosure of which is incorporated by reference). Strain GX1670 has been deposited at the USDA Northern Regional Research Laboratory, Peoria, Illinois, with accession number NRRL B-15571.

Plasmid pGX2231 is extracted from *E. coli* strain GX1670 by the method of Holmes and Quigley (Anal. Biochem., 114:193-197 ([1981]). Using the procedure of Norris et al., *supra*, a BclI restriction site is introduced just upstream of the Δ2-prorennin coding region by inserting the hexanucleotide TGA TCA as follows:

```
                      TGATCA
                        ↓
. . . TTC  GAC  CAG | ATC  ACC  AGG . . .
                    | ile  thr  arg
                    |
                    | Δ2-prorennin
                 ↓

                         BclI
                     ┌─────────┐
. . . TTC  GAC  CAT  GAT  CAG  ATC  ACC  AGG . . .
                     asp  gln  ile  thr  arg
```

The Δ2-prorennin gene containing the synthesized BclI site is cleaved with BclI and with any restriction enzyme which generates a blunt end 3' to the transcription terminator of the gene.

Plasmid pGX2134 (Example V), containing the apr[BamP] gene with the BamHI site inserted just downstream from the signal peptide coding sequence, is digested with BamHI and PvuII and the small fragment is discarded. The large fragment from cleaved pGX2134 is ligated to the BclI digested Δ2-prorennin gene. The resultant plasmid contains the complete apr[BamP] signal peptide coding region linked in-frame to the coding region for Δ2-prorennin through a sequence encoding -ala-gly-asp-gln-.

Plasmid pGX2138 (Example V), containing the npr[BamP] gene with the BamHI site inserted just downstream from the signal peptide coding sequence, is digested with BamHI and EcoRV and the small fragment is discarded. The large fragment from pGX2138 is ligated to the BclI digested Δ2-prorennin gene produced as described above. The resultant plasmid contains the complete npr[BamP] signal peptide coding region linked in-frame to the coding region for Δ2-prorennin through a sequence encoding -ala-glu-asp-gln-.

Each of the plasmids containing the apr[BamP]/Δ2-prorennin and npr[BamP]/Δ2-prorennin fusions is used to transform *B. subtilis* strain BR151 by the procedure of Chang and Cohen, *supra*, and transformants are selected for chloramphenicol resistance. Chloramphenicol resistant transformants are inoculated into culture media and grown overnight. The presence of secreted proteins containing the Δ2-prorennin sequence is confirmed by Western analysis. The secreted protein, which can be recovered from the medium by conventional procedures, is capable of being autocatalytically cleaved to produce active rennin by the procedure of U.S. Patent Application Serial No. 528,421.

EV 145-231

Claims

1. A replicable plasmidic expression vector capable of directing expression and secretion of a polypeptide in a bacillus transformed therewith, which comprises:

(a) a replicon;

(b) a DNA sequence comprising the promotor and regulatory regions which control expression and secretion of a bacillus protease; and

(c) a DNA sequence encoding the amino acid sequence of a polypeptide, operably linked to said promotor and regulatory region.

2. A replicable plasmidic expression vector as claimed in claim 1 wherein the DNA sequence comprising the said promotor and regulatory regions are derived from B. amyloliquefaciens.

3. A replicable plasmidic expression vector as claimed in claim 1 or claim 2 wherein the polypeptide which is encoded is the protease whose expression and secretion in its cell of origin is controlled by the said promotor and regulatory regions.

4. A replicable plasmidic expression vector as claimed in claim 1 or claim 2 wherein the polypeptide which is encoded is a heterologous polypeptide whose expression and secretion in its cell or origin is not controlled by the said protease promotor and regulatory regions selected.

5. A replicable plasmidic expression vector as claimed in any of claims 1, 2 or 3 wherein the encoding DNA sequence is that coding for a protease derived from B. amyloliquefaciens.

6. A replicable plasmidic expression vector as claimed in claim 1 or claim 4 wherein the strucutural gene which is fused to the promotor and regulatory region comprises a gene encoding a protein having prorennin activity.

7. A replicable plasmidic expression vector as claimed in claim 1 or claim 4, wherein the structural gene which is fused to the promotor and regulatory region comprises a gene encoding a protein having protein A activity.

8. A vector comprising a replicable plasmid containing the promotor and regulatory region of a gene selected from apr[BamP] and npr[BamP] and a sequence of deoxynucleotides, adjacent the 3' end of the signal peptide coding sequence, which comprise an endonuclease restriction site not naturally present in apr[BamP] or npr[BamP].

9. A plasmid selected from the group consisting of pGx 2109, pGx 2110, pGx 2111, pGx 2134 and pGx 2138.

10. A bacillus transformed by a vector or plasmid as claimed in any of the preceding claims which is different from the bacillus from which the promotor and regulatory DNA sequence is taken.

11. B. subtilis transformed by a vector as claimed in any of claims 1 to 10.

12. A method for producing a microorganism for the expression of a polypeptide which comprises transforming a Bacillus with vector as claimed in claim 1.

13. A method of producing a polypeptide which comprises:

(a) transforming a bacillus with a replicable plasmidic expression vector as claimed in any of claims 1 to 10;

(b) growing the transformed bacilli in a culture medium and subjecting them to conditions under which the polypeptide is expressed and secreted into the medium; and

(c) recovering the polypeptide from the medium.

0133756

1/6

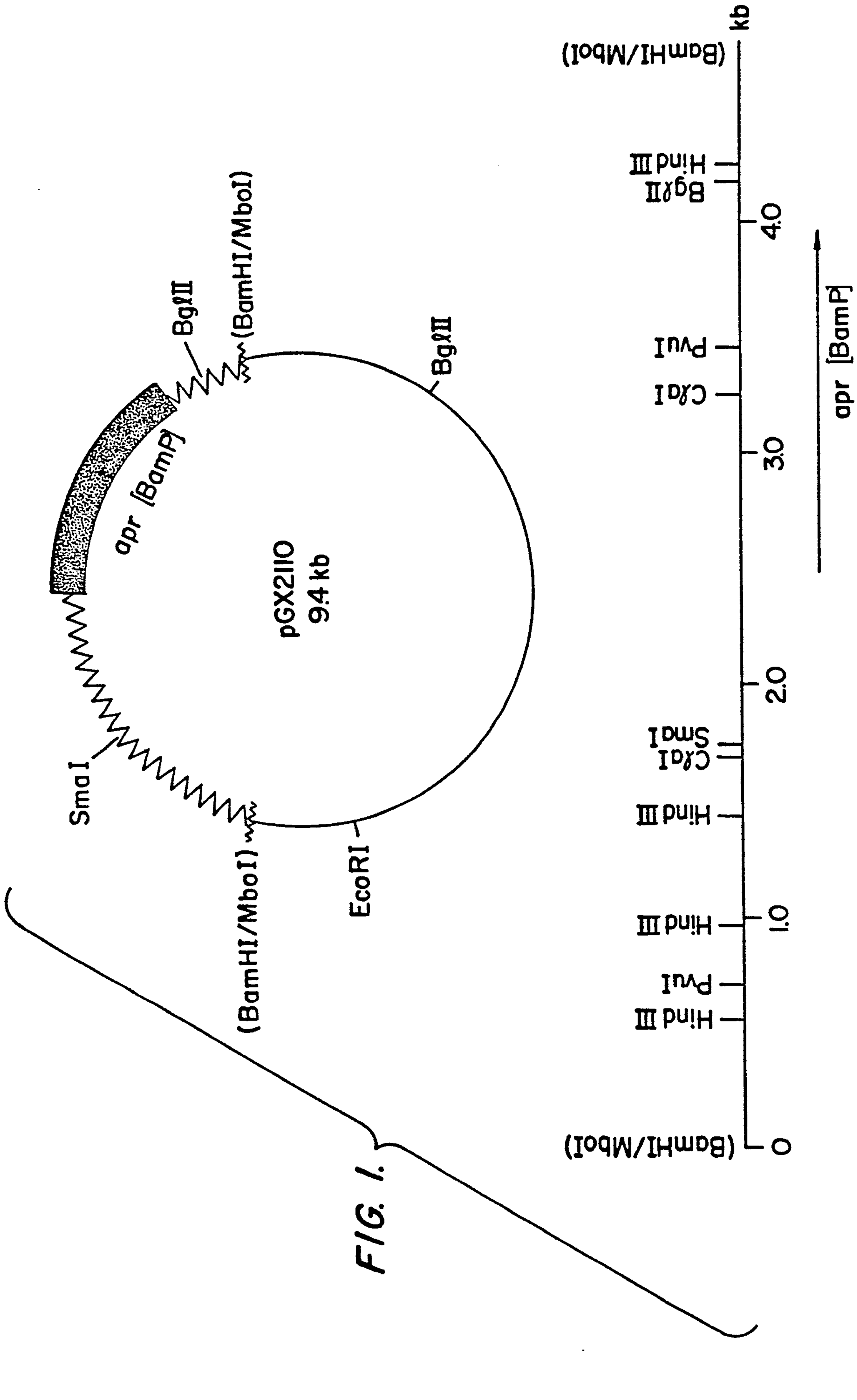


FIG. 1.

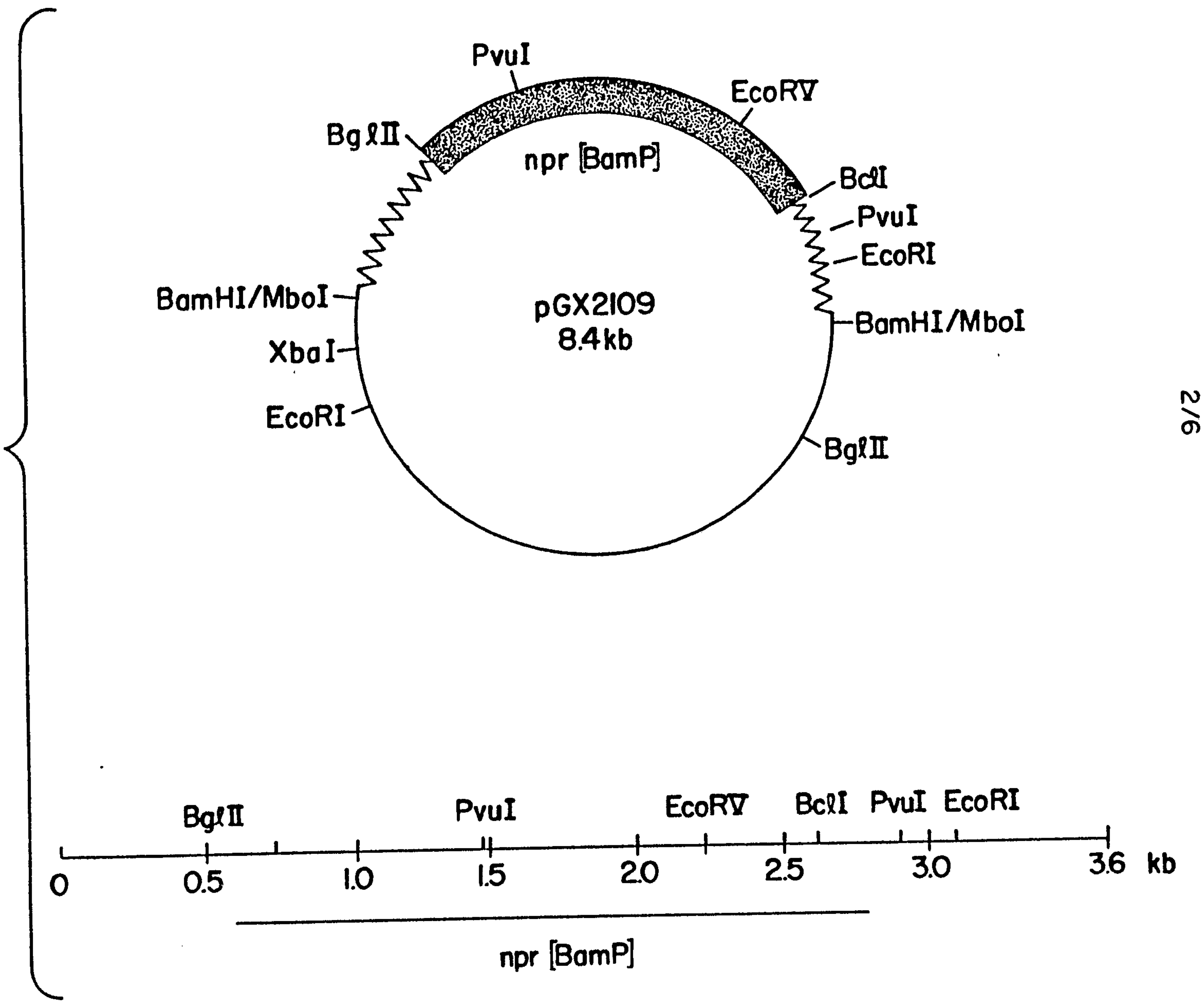

PvuI
EcoRV
BglII
npr [BamP]
BclI
PvuI
EcoRI
BamHI/MboI
pGX2109
8.4kb
BamHI/MboI
XbaI
EcoRI
BglII
BglII
PvuI
EcoRV
BclI
PvuI
EcoRI
0
0.5
1.0
1.5
2.0
2.5
3.0
3.6 kb
npr [BamP]


FIG. 2.

FIG. 3.

```
TTTTCCGCAATTATATCATTGACAATATCAACATCAATGATATTCATTATCATTATTTTTATAAAATGGTTTCACAGCTTTTCTCGGTCAAGAAAGCCA 100
AACACTGATTTCGCTTACGTTTCCATCAGTCTTCTGTATTCAACAAAAGATGACATTTATCCTGTTTTTGGAACAACCCCCAAAAATGCAAACAAACCGT 200
TCGACCCAGGAAACAAGCGAGTGATTCCTCCTGTGTACATTTACTCATGTCCATCCATCGGTTTTTTCCATTAAAATTTAAATATTTCCAGTTCCTACGA 300
AACGAAAGAGAGATGATATACCTAAATAGAAATAAAACAATCTGAAAAAAATTGGGTCTACTAAAATATTATTCCATACTATACAATTAATACACAGAAT 400
                                                 -107                  -100
AATCTGTCTATTGGTTATTCTGCAAATGAAAAAAAGGAGAGGATAAAGA GTG AGA GGC AAA AAA GTA TGG ATC AGT TTG CTG TTT 485
                                                 fmet Arg Gly Lys Lys Val Trp Ile Ser Leu Leu Phe
                                                                     PRO ------------------->
GCT TTA GCG TTA ATC TTT ACG ATG GCG TTC GGC AGC ACA TCC TCT GCC CAG GCG}GCA GGG AAA TCA AAC GGG GAA 560
Ala Leu Ala Leu Ile Phe Thr Met Ala Phe Gly Ser Thr Ser Ser Ala Gln Ala}Ala Gly Lys Ser Asn Gly Glu
                                                                            -50
AAG AAA TAT ATT GTC GGG TTT AAA CAG ACA ATG AGC ACG ATG AGC GCC GCT AAG AAG AAA GAT GTC ATT TCT GAA 635
Lys Lys Tyr Ile Val Gly Phe Lys Gln Thr Met Ser Thr Met Ser Ala Ala Lys Lys Lys Asp Val Ile Ser Glu

AAA GGC GGG AAA GTG CAA AAG CAA TTC AAA TAT GTA GAC GCA GCT TCA GCT ACA TTA AAC GAA AAA GCT GTA AAA 710
Lys Gly Gly Lys Val Gln Lys Gln Phe Lys Tyr Val Asp Ala Ala Ser Ala Thr Leu Asn Glu Lys Ala Val Lys
                                                                                Mature ------->
GAA TTG AAA AAA GAC CCG AGC GTC GCT TAC GTT GAA GAA GAT CAC GTA GCA CAT GCG TAC|GCG CAG TCC GTG CCT 785
Glu Leu Lys Lys Asp Pro Ser Val Ala Tyr Val Glu Glu Asp His Val Ala His Ala Tyr|Ala Gln Ser Val Pro

TAC GGC GTA TCA CAA ATT AAA GCC CCT GCT CTG CAC TCT CAA GGC TAC ACT GGA TCA AAT GTT AAA GTA GCG GTT 860
Tyr Gly Val Ser Gln Ile Lys Ala Pro Ala Leu His Ser Gln Gly Tyr Thr Gly Ser Asn Val Lys Val Ala Val
                  ClaI                                                     50
ATC GAC AGC GGT ATC GAT TCT TCT CAT CCT GAT TTA AAG GTA GCA GGC GGA GCC AGC ATG GTT CCT TCT GAA ACA 935
Ile Asp Ser Gly Ile Asp Ser Ser His Pro Asp Leu Lys Val Ala Gly Gly Ala Ser Met Val Pro Ser Glu Thr

AAT CCT TTC CAA GAC AAC AAC TCT CAC GGA ACT CAC GTT GCC GGC ACA GTT GCG GCT CTT AAT AAC TCA ATC GGT 1010
Asn Pro Phe Gln Asp Asn Asn Ser His Gly Thr His Val Ala Gly Thr Val Ala Ala Leu Asn Asn Ser Ile Gly
                                                                           100
GTA TTA GGC GTT GCG CCA AGC GCA TCA CTT TAC GCT GTA AAA GTT CTC GGT GCT GAC GGT TCC GGC CAA TAC AGC 1085
Val Leu Gly Val Ala Pro Ser Ala Ser Leu Tyr Ala Val Lys Val Leu Gly Ala Asp Gly Ser Gly Gln Tur Ser
                                PvuI
TGG ATC ATT AAC GGA ATC GAG TGG GCG ATC GCA AAC AAT ATG GAC GTT ATT AAC ATG AGC CTC GGC GGA CCT TCT 1160
Trp Ile Ile Asn Gly Ile Glu Trp Ala Ile Ala Asn Asn Met Asp Val Ile Asn Met Ser Leu Gly Gly Pro Ser
                                                                           150
GGT TCT GCT GCT TTA AAA GCG GCA GTT GAT AAA GCC GTT GCA TCC GGC GTC GTA GTC GTT GCG GCA GCC GGT AAC 1235
Gly Ser Ala Ala Leu Lus Ala Ala Val Asp Lys Ala Val Ala Ser Gly Val Val Val Val Ala Ala Ala Gly Asn

GAA GGC ACT TCC GGC AGC TCA AGC ACA GTG GGC TAC CCT GGT AAA TAC CCT TCT GTC ATT GCA GTA GGC GCT GTT 1300
Glu Gly Thr Ser Gly Ser Ser Ser Thr Val Gly Tyr Pro Gly Lys Tyr Pro Ser Val Ile Ala Val Gly Ala Val
                                                                           200
GAC AGC AGC AAC CAA AGA GCA TCT TTC TCA AGC GTA GGA CCT GAG CTT GAT GTC ATG GCA CCT GGC GTA TCT ATC 1375
Asp Ser Ser Asn Gln Arg Ala Ser Phe Ser Ser Val Gly Pro Glu Leu Asp Val Met Ala Pro Gly Val Ser Ile

CAA AGC ACG CTT CCT GGA AAC AAA TAC GGG GCG TAC AAC GGT ACG TCA ATG GCA TCT CCG CAC GTT GCC GGA GCG 1450
Gln Ser Thr Leu Pro Gly Asn Lys Tyr Gly Ala Tyr Asn Gly Thr Ser Met Ala Ser Pro His Val Ala Gly Ala
                                                                           250
GCT GCT TTG ATT CTT TCT AAG CAC CCG AAC TGG ACA AAC ACT CAA GTC CGC AGC AGT TTA GAA AAC ACC ACT ACA 1525
Ala Ala Leu Ile Leu Ser Lus His Pro Asn Trp Thr Asn Thr Gln Val Arg Ser Ser Leu Glu Asn Thr Thr Thr
                                                                       275
AAA CTT GGT GAT TCT TTC TAC TAT GGA AAA GGG CTG ATC AAC GTA CAG GCG GCA GCT CAG TAA AACATAAAAAACCGGC 1604
Lys Leu Gly Asp Ser Phe Tyr Tyr Gly Lys Gly Leu Ile Asn Val Gln Ala Ala Ala Gln ***

CTTGGCCCCGCCGGTTTTTTATTATTTTTCTTCCTCCGCATGTTCAATCCGCTCCATAATCGACGGATGGCTCCCTCTGAAAATTTTAACGAGAAACGGC 1704
GGGTTGACCCGGCTCAGTCCCGTAACGGCCAAGTCCTGAAACGTCTCAATCGCCGCTTCCCGGTTTCCGGTCAGCTCAATGCCGTAACGGTCGGCGGCGT 1804
TTTCCTGATACCGGGAGACGGCATTCGTAATCGGATCAGAAGCAAAACTGAGCA 1854
```

GATCTTAACA TTTTTCCCCT ATCATTTTTC CCGTCTTCAT TTGTCATTTT TTCCAGAAAA AATCGCGTCA TTCGACTCAT GTCTAATCCA ACACGTGTCT 100

CTCGGCTTAT CCCCTGACAC CGCCCGCCGA CAGCCCGCAT GGGACGATTC TATCAATTCA GCCGCCGAGT CTAGTTTTAT ATTGCAGAAT GCGAGATTGC 200

-221
TGGTTTATTA TAACAATATA AGTTTTCATT ATTTTCAAAA AGGGGGATTT ATT GTG GGT TTA GGT AAG AAA TTG TCT GTT GCT GTC GCC GCT 292
fmet Gly Leu Gly Lys Lys Leu Ser Val Ala Val Ala Ala

-200 PRO
TCC TTT ATG AGT TTA ACC ATC AGT CTG CCG GGT GTT CAG GCC}GCT GAG AAT CCT CAG CTT AAA GAA AAC CTG ACG AAT TTT GTA 376
Ser Phe Met Ser Leu Thr Ile Ser Leu Pro Gly Val Gln Ala}Ala Glu Asn Pro Gln Leu Lys Glu Asn Leu Thr Asn Phe Val

CCG AAG CAT TCT TTG GTG CAA TCA GAA TTG CCT TCT GTC AGT GAC AAA [illegible]C AAG CAA TAC TTG AAA CAA AAC GGC AAA GTC 460
Pro Lys His Ser Leu Val Gln Ser Glu Leu Pro Ser Val Ser Asp Lys A[illegible]le Lys Gln Tyr Leu Lys Gln Asn Gly Lys Val

-150
TTT AAA GGC AAT CCT TCT GAA AGA TTG AAG CTG ATT GAC CAA ACG ACC GAT GAT CTC GGC TAC AAG CAC TTC CGT TAT GTG CCT 544
Phe Lys Gly Asn Pro Ser Glu Arg Leu Lys Leu Ile Asp Gln Thr Thr Asp Asp Leu Gly Tyr Lys His Phe Arg Tyr Val Pro

-100
GTC GTA AAC GGT GTG CCT GTG AAA GAC TCT CAA GTC ATT ATT CAC GTC GAT AAA TCC AAC AAC GTC TAT GCG ATT AAC GGT GAA 628
Val Val Asn Gly Val Pro Val Lys Asp Ser Gln Val Ile Ile His Val Asp Lys Ser Asn Asn Val Tyr Ala Ile Asn Gly Glu

PvuI
TTA AAC AAC GAT GTT TCC GCC AAA ACG GCA AAC AGC AAA AAA TTA TCT GCA AAT CAG GCG CTG GAT CAT GCT TAT AAA GCG ATC 712
Leu Asn Asn Asp Val Ser Ala Lys Thr Ala Asn Ser Lys Lys Leu Ser Ala Asn Gln Ala Leu Asp His Ala Tyr Lys Ala Ile

-50
GGC AAA TCA CCT GAA GCC GTT TCT AAC GGA ACC GTT GCA AAC AAA AAC AAA GCC GAG CTG AAA GCA GCA GCC ACA AAA GAC GGC 796
Gly Lys Ser Pro Glu Ala Val Ser Asn Gly Thr Val Ala Asn Lys Asn Lys Ala Glu Leu Lys Ala Ala Ala Thr Lys Asp Gly

AAA TAC CGC CTC GCC TAT GAT GTA ACC ATC CGC TAC ATC GAA CCG GAA CCT GCA AAC TGG GAA GTA ACC GTT GAT GCG GAA ACA 880
Lys Tyr Arg Leu Ala Tyr Asp Val Thr Ile Arg Tyr Ile Glu Pro Glu Pro Ala Asn Trp Glu Val Thr Val Asp Ala Glu Thr

Mature
GGA AAA ATC CTG AAA AAG CAA AAC AAA GTG GAG CAT}GCC GCC ACA ACC GGA ACA GGT ACG ACT CTT AAA GGA AAA ACG GTC TCA 964
Gly Lys Ile Leu Lys Lys Gln Asn Lys Val Glu His}Ala Ala Thr Thr Gly Thr Gly Thr Thr Leu Lys Gly Lys Thr Val Ser

TTA AAT ATT TCT TCT GAA AGC GGC AAA TAT GTG CTG CGC GAT CTT TCT AAA CCT ACC GGA ACA CAA ATT ATT ACG TAC GAT CTG 1048
Leu Asn Ile Ser Ser Glu Ser Gly Lys Tyr Val Leu Arg Asp Leu Ser Lys Pro Thr Gly Thr Gln Ile Ile Thr Tyr Asp Leu

50
CAA AAC CGC GAG TAT AAC CTG CCG GGC ACA CTC GTA TCC AGC ACC ACA AAC CAG TTT ACA ACT TCT TCT CAG CGC GCT GCC GTT 1132
Gln Asn Arg Glu Tyr Asn Leu Pro Gly Thr Leu Val Ser Ser Thr Thr Asn Gln Phe Thr Thr Ser Ser Gln Arg Ala Ala Val

100
GAT GCG CAT TAC AAC CTC GGC AAA GTG TAT GAT TAT TTC TAT CAG AAG TTT AAT CGC AAC AGC TAC GAC AAT AAA GGC GGC AAG 1216
Asp Ala His Tyr Asn Leu Gly Lys Val Tyr Asp Tyr Phe Tyr Gln Lys Phe Asn Arg Asn Ser Tyr Asp Asn Lys Gly Gly Lys

ATC GTA TCC TCC GTT CAT TAC GGC AGC AGA TAC AAT AAC GCA GCC TGG ATC GGC GAC CAA ATG ATT TAC GGT GAC GGC GAC GGT 1300
Ile Val Ser Ser Val His Tyr Gly Ser Arg Tyr Asn Asn Ala Ala Trp Ile Gly Asp Gln Met Ile Tyr Gly Asp Gly Asp Gly

150
TCA TTC TTC TCA CCT CTT TCC GGT TCA ATG GAC GTA ACC GCT CAT GAA ATG ACA CAT GGC GTT ACA CAG GAA ACA GCC AAC CTG 1384
Ser Phe Phe Ser Pro Leu Ser Gly Ser Met Asp Val Thr Ala His Glu Met Thr His Gly Val Thr Gln Glu Thr Ala Asn Leu

AAC TAC GAA AAT CAG CCG GGC GCT TTA AAC GAA TCC TTC TCT GAT GTA TTC GGG TAC TTC AAC GAT ACT GAG GAC TGG GAT ATC 1468
Asn Tyr Glu Asn Gln Pro Gly Ala Leu Asn Glu Ser Phe Ser Asp Val Phe Gly Tyr Phe Asn Asp Thr Glu Asp Trp Asp Ile

200
GGT GAA GAT ATT ACG GTC AGC CAG CCG GCT CTC CGC AGC TTA TCC AAT CCG ACA AAA TAC GGA CAG CCT GAT AAT TTC AAA AAT 1552
Gly Glu Asp Ile Thr Val Ser Gln Pro Ala Leu Arg Ser Leu Ser Asn Pro Thr Lys Tyr Gly Gln Pro Asp Asn Phe Lys Asn

TAC AAA AAC CTT CCG AAC ACT GAT GCC GGC GAC TAC GGC GGC GTG CAT ACA AAC AGC GGA ATC CCG AAC AAA GCC GCT TAC AAT 1636
Tyr Lys Asn Leu Pro Asn Thr Asp Ala Gly Asp Tyr Gly Gly Val His Thr Asn Ser Gly Ile Pro Asn Lys Ala Ala Tyr Asn

250
ACG ATT ACA AAA ATC GGC GTG AAC AAA GCG GAG CAG ATT TAC TAT CGT GCT CTG ACG GTA TAC CTC ACT CCG TCA TCA ACT TTT 1720
Thr Ile Thr Lys Ile Gly Val Asn Lys Ala Glu Gln Ile Tyr Tyr Arg Ala Leu Thr Val Tyr Leu Thr Pro Ser Ser Thr Phe

AAA GAT GCA AAA GCC GCT TTG ATT CAA TCT GCG CGG GAC CTT TAC GGC TCT CAA GAT GCT GCA AGC GTA GAA GCT GCC TGG AAT 1804
Lys Asp Ala Lys Ala Ala Leu Ile Gln Ser Ala Arg Asp Leu Tyr Gly Ser Gln Asp Ala Ala Ser Val Glu Ala Ala Trp Asn

300 BclI
GCA GTC GGA TTG TAA ACAAGAAAAGAGACCGGAAATCCGGTCTCTTTTTTATATCTAAAAACATTTCACAGTGGCTTCACCATGATCATATATGTCTTTTCCCG 1908
Ala Val Gly Leu ***

FIG. 4.

FIG. 5.

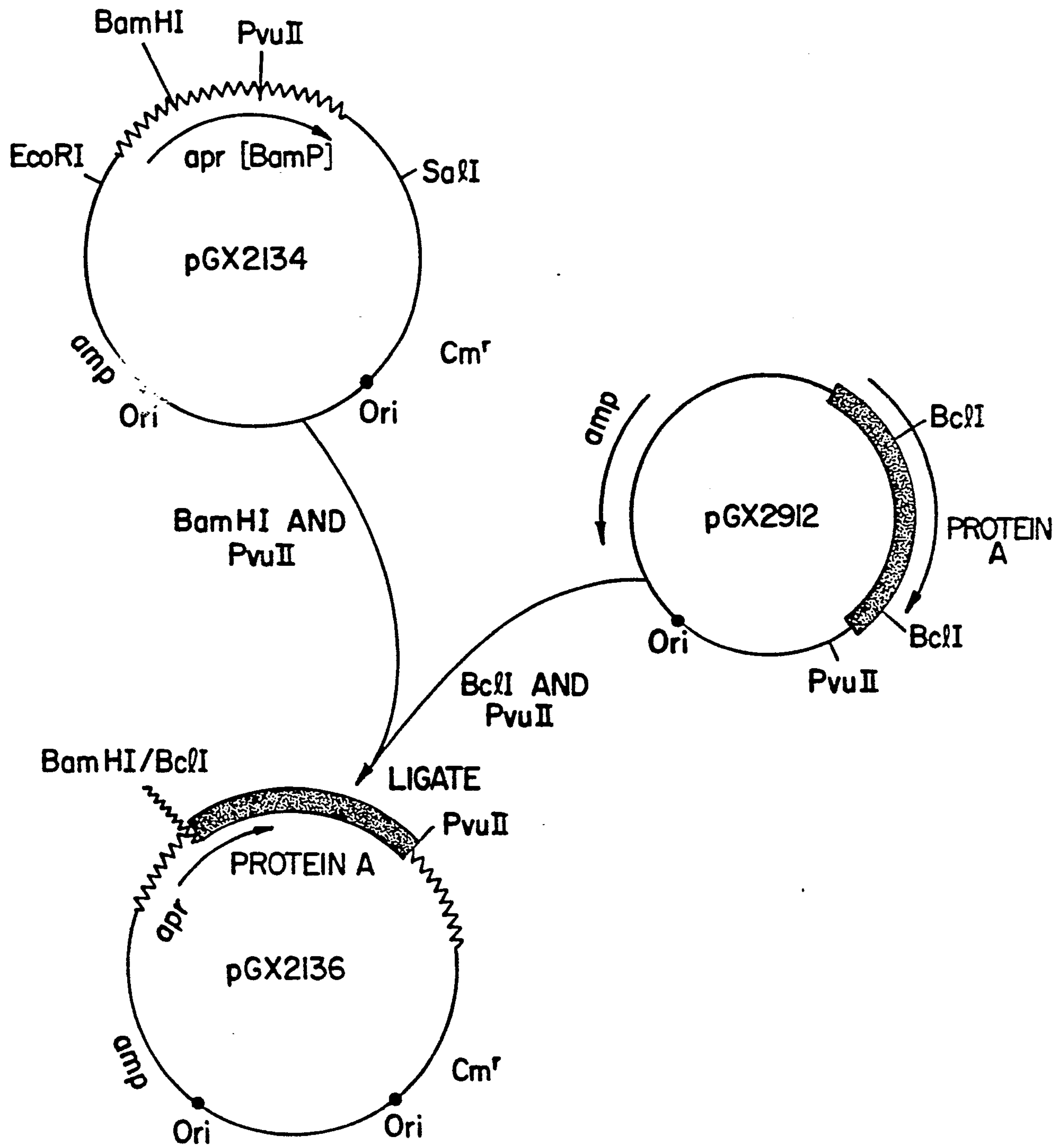
BamHI
PvuII
EcoRI
apr [BamP]
SalI
pGX2134
amp
Cmr
Ori
Ori
BamHI AND
PvuII
amp
BclI
pGX2912
PROTEIN
A
Ori
BclI
PvuII
BclI AND
PvuII
BamHI/BclI
LIGATE
PvuII
PROTEIN A
apr
pGX2136
amp
Cmr
Ori
Ori

*FIG. 6.*

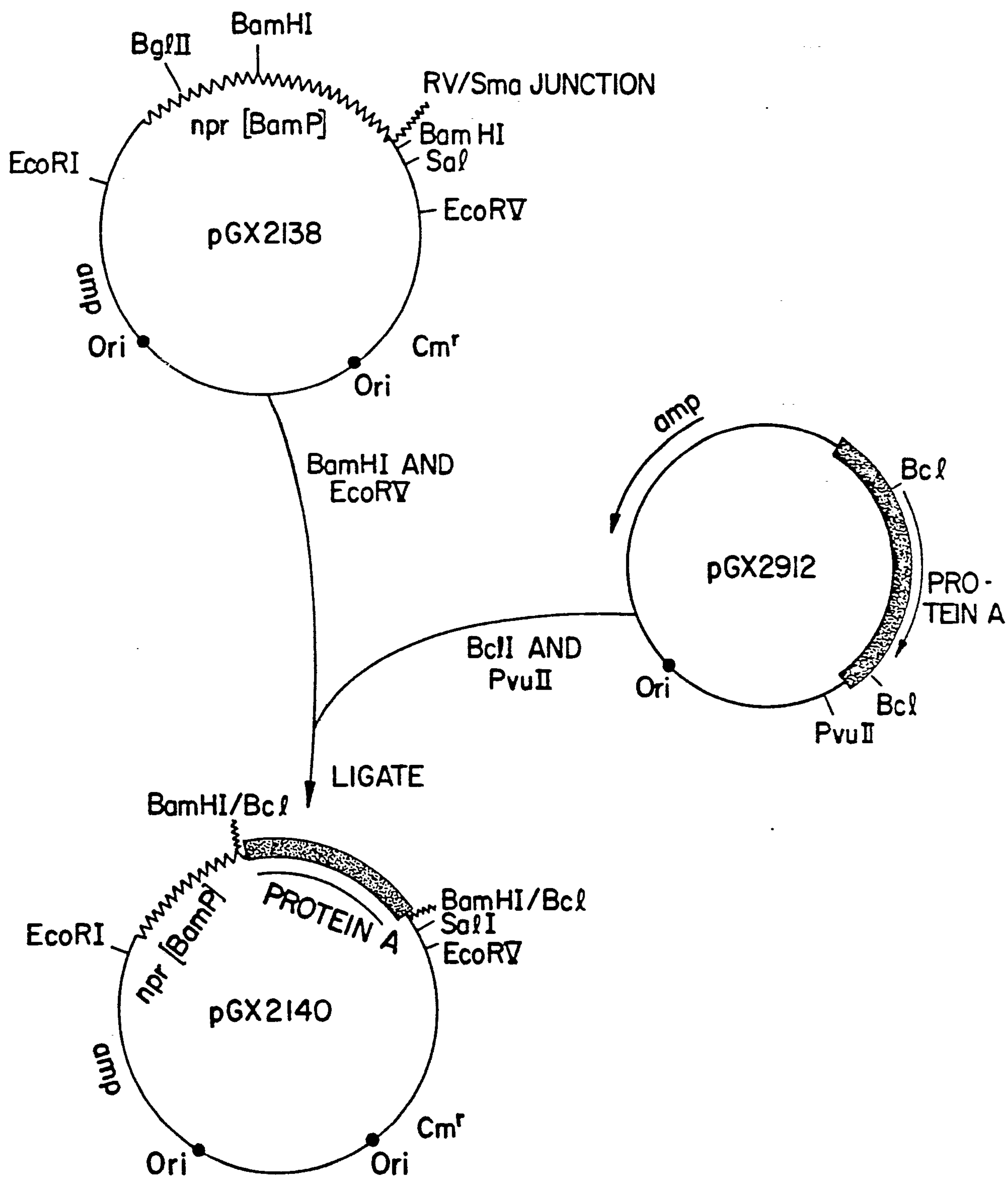
BamHI
BglII
RV/Sma JUNCTION
npr [BamP]
Bam HI
Saℓ
EcoRI
EcoRV
pGX2138
amp
Ori
Cmʳ
Ori
BamHI AND
EcoRV
amp
Bcℓ
pGX2912
PRO-
TEIN A
BclI AND
PvuII
Ori
Bcℓ
PvuII
LIGATE
BamHI/Bcℓ
PROTEIN A
BamHI/Bcℓ
SalI
EcoRI
npr [BamP]
EcoRV
pGX2140
amp
Cmʳ
Ori
Ori